# EUROPEAN PATENT APPLICATION

(11) **EP 3 167 833 A1**
(43) Date of publication of application: **17.05.2017**
(21) Application number: 15818681.7
(22) Date of filing: 18.06.2015
(51) Int. Cl.: A61B 18/12, A61B 18/14

(54) **ENDOSCOPIC SUBMUCOSAL DISSECTION DEVICE AND ENDOSCOPE SYSTEM**

(30) Priority: 10.07.2014 JP 2014142194
(71) Applicant: Olympus Corporation, Hachioji-shi Tokyo 192-8507 (JP)
(72) Inventor: MIKKAICHI Takayasu, Tokyo 192-8507 (JP)
(74) Representative: Schicker, Silvia
(86) International application number: PCT/JP2015/067587
(87) International publication number: WO 2016/006407

(57) **Abstract**

An endoscopic submucosal dissection device includes: an attachment/detachment portion that is separably attached to an insertion portion of an endoscope having an observation portion provided at the insertion portion to observe a front side; a mucous membrane support portion that supports a mucous membrane; a muscular layer support portion that supports the muscular layer; a wire portion that is formed in a linear shape from a conductive material and is disposed so that an intermediate portion in its longitudinal direction is located at a distal end side in relation to first and second ends in the longitudinal direction and the intermediate portion is located at the distal end side in relation to the insertion portion; an insulation member that exposes part of the intermediate portion of the wire portion as an electrode portion and covers an outer circumferential surface of a remaining portion of the intermediate portion of the wire portion; and an operation portion that is connected to the first and second ends of the wire portion.

## Description

### [Technical Field]

The present invention relates to an endoscopic submucosal dissection device and an endoscope system.

Priority is claimed on Japanese Patent Application No. 2014-142194, filed July 10, 2014, the content of which is incorporated herein by reference.

### [Background Art]

In recent years, when a tissue is dissected in endoscopic submucosal dissection (hereinafter, referred to as "ESD"), for example, as disclosed in Patent Literature 1, a high-frequency treatment tool is inserted through a channel formed in an insertion portion of an endoscope and a tissue is dissected by the high-frequency treatment tool.

The high-frequency treatment tool disclosed in Patent Literature 1 includes a sheath, a pair of arm portions disposed inside the sheath to be movable forward and backward, a linear treatment electrode connecting distal ends of the pair of arm portions to each other, an operation wire disposed inside the sheath to be movable forward and backward, and an operation portion connected to base ends of the sheath and the operation wire.

The pair of arm portions are closed when the pair or arm portions are retracted inside the sheath. On the other hand, the pair or arm portions are opened in a direction moving away from a center axis line of the sheath when the pair of arm portions are projected from a distal end of the sheath. When the pair of arm portions are opened, the treatment electrode is stretched between the arm portions.

Next, a technique of separating a submucosal layer and dissecting a lesion formed on a surface of an alimentary canal by the high-frequency treatment tool with the above-described configuration will be described.

A physiological saline is injected into the submucosal layer so that the lesion part protrudes.

The high-frequency treatment tool is inserted into the channel of an endoscope and the pair of arm portions are projected from a distal end of the channel. When the operation portion is operated so that the operation wire is moved forward with respect to the sheath, the pair of arm portions are separated from each other so that a straight portion is formed in the treatment electrode. A high-frequency current is supplied to the treatment electrode while the straight portion comes into contact with the submucosal layer. The submucosal layer is dissected by the straight portion so that the lesion part is separated.

### [Citation List]

### [Patent Literature]

[Patent Literature 1]
Japanese Unexamined Patent Application, First Publication No. 2007-319612

### [Summary of Invention]

### [Technical Problem]

In general, an observation portion having an imaging element for observing a front side is provided at the insertion portion of the endoscope. An image acquired by the observation portion is transmitted to a display unit and thus a user performs a treatment while checking an image displayed on the display unit.

In order to relatively move a tissue dissecting position to be dissected by the high-frequency treatment tool in a direction intersecting a center axis of the insertion portion, the insertion portion is moved in the intersecting direction. A position of a distal-end-side opening of the channel formed in the insertion portion is displaced and the distal end side of the high-frequency treatment tool inserted through the channel is also displaced in the same direction as that of the opening. In this way, the tissue dissecting position to be dissected by the high-frequency treatment tool can be displaced.

However, when the insertion portion moves, a position of the observation portion is also displaced along with the distal-end-side opening of the channel. For this reason, a field of view of an image displayed on the display unit and observed by the user moves and thus the user cannot easily perform a treatment.

The present invention is made in view of the above-described problems and an object of the present invention is to provide an endoscopic submucosal dissection device which suppresses a displacement of a field of view of an observation portion even when a tissue dissecting position is displaced in a direction intersecting a center axis of an insertion portion and an endoscope system which includes the endoscopic submucosal dissection device.

### [Solution to Problem]

In order to achieve the aforementioned objects, according to a first aspect of the present invention, there is provided an endoscopic submucosal dissection device including: an attachment/detachment portion that is separably attached to an insertion portion of an endoscope having an observation portion provided at the insertion portion to observe a front side; a mucous membrane support portion that supports a mucous membrane so that the mucous membrane does not approach a muscular layer; a muscular layer support portion that supports a muscular layer so that the muscular layer does not approach the mucous membrane; a wire portion that is formed in a linear shape from a conductive material and is disposed so that an intermediate portion in its longitudinal direction is located at a distal end side in relation to first and second end portions in the longitudinal direction and the intermediate portion is located at the distal end side in relation to the insertion portion; an insulation member that exposes part of the intermediate portion of the wire portion as an electrode portion and covers an outer circumferential surface of a remaining portion of the intermediate portion of the wire portion; and an operation portion that is connected to the first and second end portions of the wire portion.

According to a second aspect of the present invention, the endoscopic submucosal dissection device according to the first aspect may further include a holding portion that holds the wire portion to be movable forward and backward.

According to a third aspect of the present invention, an endoscope system includes: the endoscopic submucosal dissection device according to the above-described aspect; and the endoscope, wherein the intermediate portion of the wire portion and the observation portion are disposed so that the intermediate portion of the wire portion and the observation portion do not overlap each other when the attachment/detachment portion of the endoscopic submucosal dissection device is attached to the insertion portion of the endoscope and is viewed in a direction along a center axis of the insertion portion.

According to a fourth aspect of the present invention, in the endoscope system according to the third aspect, the insertion portion may be provided with an illumination portion emitting illumination light forward and the intermediate portion of the wire portion and the illumination portion may be disposed so that the intermediate portion of the wire portion and the illumination portion do not overlap each other when the attachment/detachment portion of the endoscopic submucosal dissection device is attached to the insertion portion of the endoscope and is viewed in a direction along the center axis.

### [Advantageous Effects of Invention]

According to the endoscopic submucosal dissection device and the endoscope system of the present invention, a displacement of a field of view of an observation portion can be suppressed even when a tissue dissecting position is displaced in a direction intersecting a center axis of an insertion portion.

### [Brief Description of Drawings]

Fig. 1 is an overall view showing an endoscope system according to a first embodiment of the present invention in a partially cutaway state.
Fig. 2 is a perspective view showing a distal end side of the endoscope system according to the first embodiment.
Fig. 3 is a top view showing an operation portion of an ESD device of the endoscope system according to the first embodiment.
Fig. 4 is a diagram showing an action of the endoscope system according to the first embodiment.
Fig. 5 is a front view showing the endoscope system according to the first embodiment.
Fig. 6 is a cross-sectional view showing a main part of the operation portion of the endoscope system according to the first embodiment.
Fig. 7 is a diagram showing an example of an image displayed on a display unit of the endoscope system according to the first embodiment.
Fig. 8 is a perspective view showing a distal end side of an ESD device according to a first modified example of the first embodiment.
Fig. 9 is a top view showing a distal end side of an ESD device according to a second modified example of the first embodiment.
Fig. 10 is a cross-sectional view showing a distal end side of an ESD device according to a third modified example of the first embodiment.
Fig. 11 is a top view showing a distal end side of an ESD device according to a fourth modified example of the first embodiment.
Fig. 12 is a top view showing a distal end side of an endoscope system according to a second embodiment of the present invention.
Fig. 13 is a perspective view showing the distal end side of the endoscope system according to the second embodiment.
Fig. 14 is a perspective view showing a distal end side of an endoscope system according to a modified example of the second embodiment.
Fig. 15 is a perspective view showing a distal end side of an endoscope system according to a third embodiment of the present invention.
Fig. 16 is a perspective view showing an action of the distal end side of the endoscope system according to the third embodiment.

### [Description of Embodiments]

### (First Embodiment)

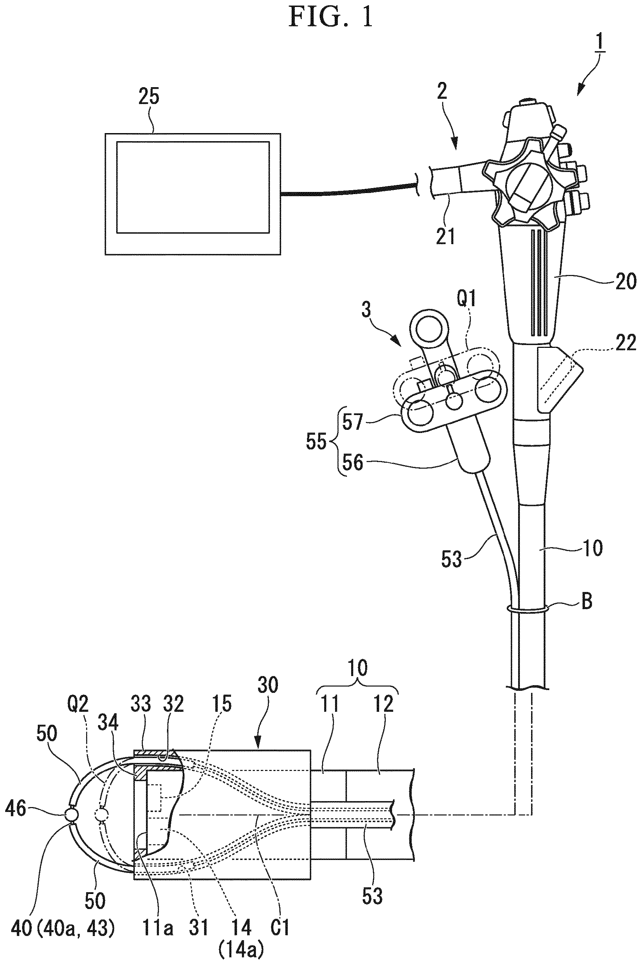

Hereinafter, a first embodiment of an endoscope system according to the present invention will be described with reference to Figs. 1 to 11.

As shown in Fig. 1, an endoscope system 1 according to the embodiment includes an endoscope 2 and an ESD device (an endoscopic submucosal dissection device) 3. The ESD device 3 is separably attached to an insertion portion 10 of the endoscope 2. Hereinafter, a position near the insertion portion 10 in an endoscope operation portion 20 to be described later will be referred to as a distal end side and a position near the endoscope operation portion 20 in the insertion portion 10 will be referred to as a base end side.

A configuration of the endoscope 2 is not particularly limited. For example, in the embodiment, the endoscope 2 includes the soft insertion portion 10 and the endoscope operation portion 20 attached to a base end of the insertion portion 10.

The insertion portion 10 is formed in a columnar shape. The insertion portion 10 includes a rigid distal end 11 which is provided at a distal end and a bending portion 12 which is provided at the base end side in relation to the rigid distal end 11. The bending portion 12 is bendable. A distal end face 11a of the rigid distal end 11 is provided with an illumination unit (an illumination portion) 14 which emits illumination light forward and an observation unit (an observation portion) 15 which observes a front side. Here, the illumination unit and the observation unit are exposed to the outside.

The illumination unit 14 includes a light guide 14a. The light guide 14a extends toward the base end side while being inserted through the insertion portion 10.

The observation unit 15 includes an imaging element such as a CMOS image sensor (not shown). A display unit 25 is connected to the endoscope operation portion 20 via a universal cable 21. An image of an observation target within a predetermined field of view acquired by the imaging element is converted into a signal and the signal is transmitted to the display unit 25.

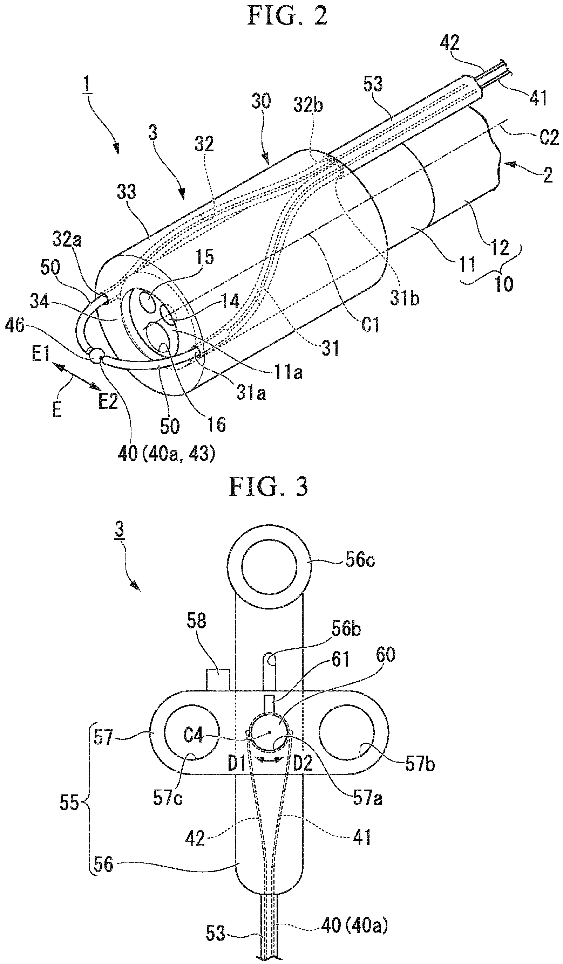

As shown in Fig. 2, a distal end of a channel 16 formed in the insertion portion 10 is opened to the distal end face 11 a of the rigid distal end 11. As shown in Fig. 1, a base end of the channel 16 communicates with a forceps opening 22 provided at the endoscope operation portion 20.

The light guide 14a passes through the universal cable 21 to be connected to a light source having a xenon lamp (not shown). When a predetermined power is supplied to the light source, light is emitted from the xenon lamp. The light is guided by the light guide 14a and is emitted as illumination light toward the front side of the distal end face 11 a of the insertion portion 10.

As shown in Figs. 1 to 3, the ESD device 3 includes a cap 30, a wire portion 40, an insulation coating (an insulation member) 50, and an operation portion 55. The wire portion 40 is inserted through penetration holes (holding portions) 31 and 32 formed in the cap 30. The insulation coating 50 covers an outer circumferential surface of the wire portion 40. The operation portion 55 is connected to a first end portion 41 and a second end portion 42 (see Fig. 2) of the wire portion 40.

As shown in Figs. 1 and 2, the cap 30 includes a cylindrical cap body 33 and a locking portion 34 provided at a distal end of an inner circumferential surface of the cap body 33.

When the cap body 33 is formed of an elastic material, an inner diameter of the cap body 33 is equal to or slightly smaller than an outer diameter of the insertion portion 10 of the endoscope 2. Further, when the cap body 33 is formed of a rigid material, the inner diameter of the cap body 33 is equal to or slightly larger than the outer diameter of the insertion portion 10 of the endoscope 2. The cap body 33 is provided with the penetration holes 31 and 32.

As shown in Fig. 2, a distal-end-side opening 31a of the penetration hole 31 and a distal-end-side opening 32a of the penetration hole 32 are formed at opposite sides with a center axis C1 of the cap body 33 interposed therebetween. On the other hand, a base-end-side opening 31b of the penetration hole 31 and a base-end-side opening 32b of the penetration hole 32 are disposed side by side to be adjacent to each other. The penetration holes 31 and 32 are formed in a spiral shape in the cap body 33.

As shown in Fig. 1, the locking portion 34 is formed in an entire circumference of the inner circumferential surface of the cap body 33 to protrude from the inner circumferential surface of the cap body 33.

The cap body 33 and the locking portion 34 are integrally formed from a transparent resin material such as ETFE (Ethylene TetraFluoroEthylene) having biocompatibility.

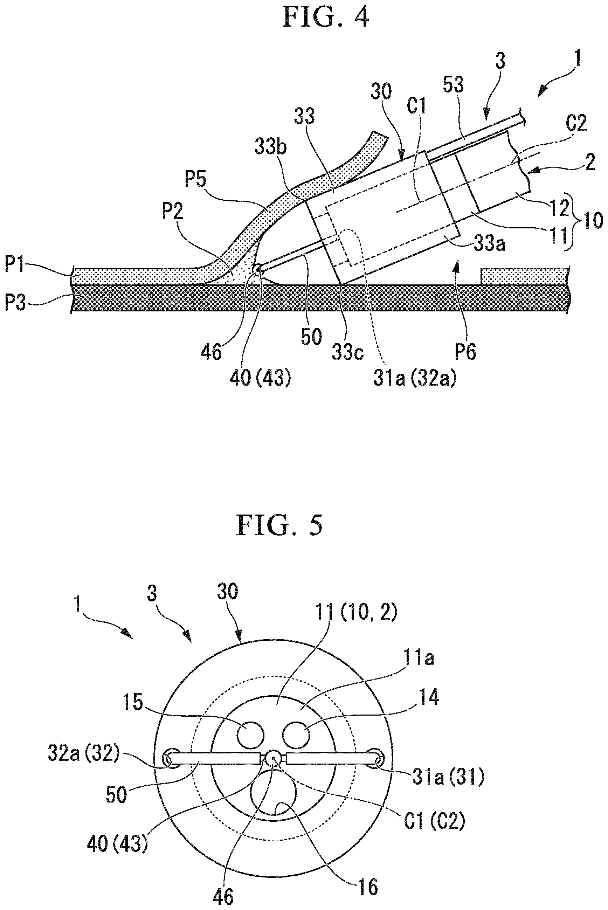

As shown in Fig. 4, a base end side of the cap body 33 is formed as an attachment/detachment portion 33a separably attached to the rigid distal end 11 of the insertion portion 10.

Although an action of the endoscope system 1 will be described later, a portion which is located between the opening 31 a of the penetration hole 31 and the opening 32a of the penetration hole 32 along an outer circumferential surface at a distal end of an outer circumferential surface of the cap body 33 forms a mucous membrane support portion 33b which supports a mucous membrane P1 so that the mucous membrane P1 does not approach a muscular layer P3. A portion which is opposite to the mucous membrane support portion 33b at the distal end of the outer circumferential surface of the cap body 33 with the center axis C1 interposed therebetween forms a muscular layer support portion 33c which supports the muscular layer P3 so that the muscular layer P3 does not approach the mucous membrane P1.

In this way, the attachment/detachment portion 33a, the mucous membrane support portion 33b, the muscular layer support portion 33c, and the penetration holes 31 and 32 are provided at the cap 30.

When the attachment/detachment portion 33a of the cap 30 is attached to the insertion portion 10, the center axis C1 of the cap body 33 matches a center axis C2 of the insertion portion 10.

As shown in Figs. 1 to 3, the wire portion 40 is obtained by bending a linear wire 40a, which is formed of a conductive material such as stainless steel or NiTi alloy, into a loop shape. More specifically, the wire portion 40 has a configuration in which an intermediate portion 43 in the longitudinal direction of the wire portion 40 is disposed at the distal end side in relation to the first end portion 41 and the second end portion 42 in the longitudinal direction of the wire portion 40. As the wire 40a, any one of a single wire and a stranded wire can be used. When the cap 30 is attached to the insertion portion 10, the intermediate portion 43 of the wire portion 40 is disposed at the distal end side in relation to the distal end face 11 a of the insertion portion 10.

Part of the intermediate portion 43 of the wire portion 40 is provided with a spherical electrode portion 46 having an outer diameter larger than that of the insulation coating 50. The electrode portion 46 can be formed of, for example, stainless steel or platinum (Pt).

An outer circumferential surface (of a remaining portion) other than a portion provided with the electrode portion 46 in the intermediate portion 43 of the wire portion 40 is covered by the insulation coating 50. The insulation coating 50 is formed of an electrical insulation material such as polyurethane resin or polyamide resin. The electrode portion 46 is exposed while not being covered by the insulation coating 50. As will be described later, the insulation coating 50 is formed in at least a range of the wire portion 40 protruding toward the distal end side in relation to the cap body 33 when the electrode portion 46 moves in an intersection direction E intersecting the center axis C1 of the cap body 33.

A portion nearer the first end portion 41 than the intermediate portion 43 of the wire portion 40 is inserted through the penetration hole 31 of the cap 30 to be movable forward and backward while the distal end side is covered by the insulation coating 50. On the other hand a portion nearer the second end portion 42 than the intermediate portion 43 of the wire portion 40 is inserted through the penetration hole 32 of the cap 30 to be movable forward and backward while the distal end side is covered by the insulation coating 50. In this way, the penetration holes 31 and 32 hold the wire portion 40 so that the wire portion is movable forward and backward. In this example, the intermediate portion 43 of the wire portion 40 is curved to protrude toward the distal end side.

The wire portion 40 which protrudes toward the base end side in relation to the openings 31b and 32b of the cap body 33 is inserted through the sheath 53 to be movable forward and backward. A distal end of the sheath 53 is fixed to a base end of the cap 30 by welding or adhesive.

A lumen of the sheath 53 communicates with the opening 31b of the penetration hole 31 and the opening 32b of the penetration hole 32. The sheath 53 is disposed to extend along the center axis C1 of the cap body 33.

As shown in Fig. 5, it is desirable that the intermediate portion 43 of the wire portion 40 does not overlap a portion exposed to the outside from the distal end face 11 a in each of the illumination unit 14 and the observation unit 15 when the cap 30 is attached to the insertion portion 10 and is viewed in the direction along the center axis C2 of the insertion portion 10.

In this example, the illumination unit 14 and the observation unit 15 are disposed at the same side of the intermediate portion 43 of the wire portion 40.

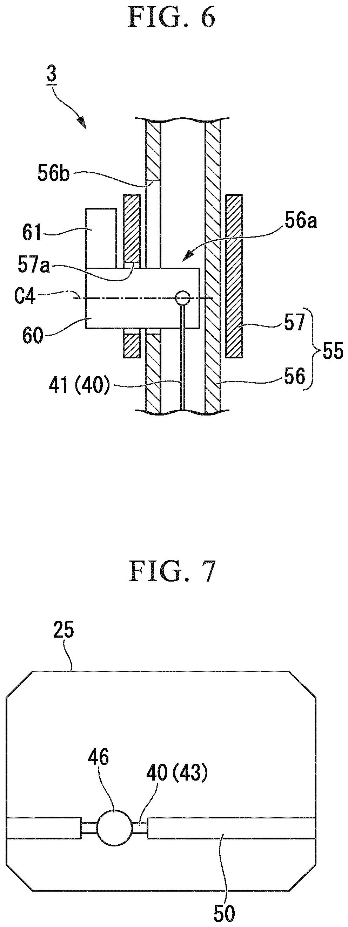

As shown in Figs. 3 and 6, the operation portion 55 includes an operation body 56 and a slider 57. The operation body 56 is fixed to a base end of the sheath 53. The slider 57 is attached to the operation body 56 to be slidable in the longitudinal direction of the sheath 53.

As shown in Fig. 6, the operation body 56 is provided with an inner space 56a. An outer surface of the operation body 56 is provided with a slit 56b which extends in the longitudinal direction of the sheath 53 and the slit 56b communicates with the inner space 56a.

A finger hook ring 56c is attached to a base end of the operation body 56.

The slider 57 is provided with a penetration hole 57a which extends in a direction orthogonal to the longitudinal direction of the sheath 53 and finger hook holes 57b and 57c which are formed with the penetration hole 57a interposed therebetween. The penetration hole 57a communicates with the inner space 56a through the slit 56b of the operation body 56. A connector 58 to and from which a cord leading to a high frequency generator (not shown) is attachable and detachable is attached to the slider 57.

A shaft member 60 is inserted through the penetration hole 57a of the slider 57 and the slit 56b of the operation body 56. The shaft member 60 is rotatable about a center axis C4 which is parallel to the above-described orthogonal direction with respect to the slider 57 and the operation body 56.

A lever 61 is attached to a portion which is exposed to the outside in the shaft member 60.

The wire portion 40 which is inserted through the sheath 53 extends inside the inner space 56a of the operation body 56 toward the base end side. The first end portion 41 and the second end portion 42 of the wire portion 40 are fixed to a portion which is disposed at the inner space 56a of the operation body 56 in the shaft member 60. The first end portion 41 and the second end portion 42 are fixed so that the shaft member 60 is interposed therebetween in the radial direction.

The first end portion 41 and the second end portion 42 are electrically connected to the connector 58 via a wire (not shown).

As shown in Fig. 1, in the ESD device 3 with the above-described configuration, when the slider 57 is moved to a position Q1 while the slider 57 is moved (pulled back) toward the base end side with respect to the operation body 56 from a state where the electrode portion 46 of the wire portion 40 protrudes from a distal end face of the cap 30, a length in which the electrode portion 46 of the wire portion 40 protrudes from the cap 30 becomes short as indicated by a position Q2.

On the other hand when the slider 57 is moved (pushed) toward the distal end side with respect to the operation body 56, a length in which the electrode portion 46 of the wire portion 40 protrudes from the cap 30 becomes long. Since the wire portion 40 is inserted through the penetration holes 31 and 32 of the cap 30, a position of the wire portion 40 is not easily displaced when the wire portion is repeatedly pushed and pulled by the operation portion 55.

When the lever 61 is gripped and the shaft member 60 is rotated about the center axis C4 toward one side (a first direction side) D1 (see Fig. 3), the first end portion 41 of the wire portion 40 is pushed inward and the second end portion 42 is pulled back so that the electrode portion 46 moves toward one side (a first intersection direction side) E1 in the intersection direction E intersecting the center axis C1 (see Fig. 2). Additionally, the intersection direction E becomes a direction intersecting the center axis C2 of the insertion portion 10 when the cap 30 is attached to the insertion portion 10.

On the other hand when the shaft member 60 is rotated about the center axis C4 toward the other side (a second direction side) D2, the second end portion 42 of the wire portion 40 is pushed inward and the first end portion 41 is pulled back so that the electrode portion 46 moves toward the other side (a second intersection direction side) E2 in the intersection direction E.

Next, an operation of cutting a mucous membrane inside a body cavity in an action of the endoscope system 1 with the above-described configuration will be described.

In advance, the slider 57 is pulled back so that a length in which the electrode portion 46 of the wire portion 40 protrudes from the cap 30 becomes short. The insertion portion 10 of the endoscope 2 is inserted from the base end side of the cap 30 and the distal end face 11 a of the insertion portion 10 is brought into contact with the locking portion 34 of the cap 30 to be positioned in the direction of the center axis C1. At this time, the positioning operation is performed so that the intermediate portion 43 of the wire portion 40 does not overlap the illumination unit 14 and the observation unit 15 when the direction of the cap 30 about the center axis C1 with respect to the insertion portion 10 is adjusted and the cap is viewed in the direction along the center axis C2.

The insertion portion 10 of the endoscope 2 and the sheath 53 of the ESD device 3 are fixed by a medical band B shown in Fig. 1.

A counter electrode plate (not shown) is mounted on a patient.

The endoscope system 1 is activated so that illumination light is emitted forward from the light guide 14a of the illumination unit 14. The observation unit 15 acquires an image of an observation target, converts the image into a signal, and transmits the signal to the display unit 25. The transmitted signal is converted into an image by a processor (not shown) and the image is displayed on the display unit 25.

As shown in Fig. 7, images of the electrode portion 46, the wire portion 40, and the insulation coating 50 are displayed on the image displayed on the display unit 25. Since the intermediate portion 43 of the wire portion 40 and the illumination unit 14 do not overlap each other when viewed in the direction along the center axis C2, the intermediate portion 43 of the wire portion 40 does not disturb the illumination light emitted forward from the illumination unit 14.

Further, since the intermediate portion 43 of the wire portion 40 and the observation unit 15 do not overlap each other when viewed in the direction along the center axis C2, the intermediate portion 43 of the wire portion 40 does not disturb an operation of acquiring a forward image. In other words, the intermediate portion 43 of the wire portion 40 does not disturb an operation of observing the image displayed on the display unit 25.

An operator inserts the insertion portion 10 of the endoscope 2 from a mouth of a patient while checking an image displayed on the display unit 25. The distal end face 11 a of the insertion portion 10 is moved to face a lesion mucous membrane part which is a target part to be dissected.

An injection needle (not shown) is inserted from the forceps opening 22 of the endoscope 2 to the channel 16. A physiological salt solution is injected to a submucosal layer of the lesion mucous membrane part by the injection needle so that the lesion mucous membrane part protrudes.

The injection needle is pulled out from the channel 16 of the endoscope 2.

The operator grips the operation portion 55 by inserting their thumb into the finger hook ring 56c of the operation body 56 and respectively inserting an index finger and a middle finger into the finger hook holes 57b and 57c of the slider 57. The slider 57 is pushed inward so that a length in which the electrode portion 46 of the wire portion 40 protrudes from the cap 30 becomes long.

A cord of a high frequency generator is attached to the connector 58. By the high frequency generator, a high-frequency voltage is applied to the electrode portion 46 via the connector 58, the wire, and the wire portion 40.

The electrode portion 46 to which the high-frequency voltage is applied is heated to about 100°C or more.

The operator pushes the endoscope system 1 so that the electrode portion 46 is pushed against the mucous membrane P1 around a lesion mucous membrane part P5 shown in Fig. 4. By the heat of the electrode portion 46, the mucous membrane P1 located around the lesion mucous membrane part P5 and pushed by the electrode portion 46 is dissected. A part to which a high-frequency voltage is applied is only the electrode portion 46 of the wire portion 40, because a portion other than the electrode portion 46 in the wire portion 40 is covered by the insulation coating 50. For this reason, since only the electrode portion 46 contacts the mucous membrane P1, the mucous membrane P1 can be easily dissected by the electrode portion 46.

When the operator rotates the shaft member 60 about the center axis C4 toward one side D1 or the other side D2 by holding the lever 61, the electrode portion 46 moves to one side E1 or the other side E2 in the intersection direction E.

When the operator adjusts a position of the electrode portion 46 in the intersection direction E by the lever 61, the mucous membrane P1 contacting the electrode portion 46 is dissected. When a predetermined range of the mucous membrane P1 around the lesion mucous membrane part P5 is dissected, a slit P6 is formed.

The cap 30 and the insertion portion 10 are inserted between the mucous membrane P1 and the muscular layer P3 through the slit P6. At this time, the mucous membrane support portion 33b of the cap 30 supports the mucous membrane P1 so that the mucous membrane P1 does not approach the muscular layer P3. On the other hand, the muscular layer support portion 33c of the cap 30 supports the muscular layer P3 so that the muscular layer P3 does not approach the mucous membrane P1.

The endoscope system 1 is pushed so that the electrode portion 46 is pushed against the submucosal layer P2 and the submucosal layer P2 is dissected by the electrode portion 46. When the operator rotates the shaft member 60 toward one side D1 or the other side D2 so that the electrode portion 46 is moved to one side E1 or the other side E2, the lesion mucous membrane part P5 is gradually separated. Since the insertion portion 10 of the endoscope 2 is not moved when the electrode portion 46 is moved, a problem in which an image displayed on the display unit 25 is moved by the displacement of the field of view of the observation unit 15 can be prevented.

After the entire lesion mucous membrane part P5 is cut and separated, the operator inserts a grip forceps (not shown) into the channel 16 of the endoscope 2. The operator endoscopically operates the grip forceps to extract the lesion mucous membrane part P5 and ends a sequence of treatment.

As described above, according to the ESD device 3 and the endoscope system 1 of the embodiment, the electrode portion 46 of the wire portion 40 moves to one side E1 or the other side E2 in the intersection direction E by the operation of the operation portion 55. Thus, a problem in which the field of view of the observation unit 15 is moved by the displacement of the insertion portion 10 of the endoscope 2 can be suppressed.

In order to move the electrode portion 46 in the intersection direction E, there is no need to bend the bending portion 12 of the insertion portion 10 or move the cap 30 along with the insertion portion 10 of the endoscope 2. For this reason, even a person who is unfamiliar with the operation of the endoscope 2 can reliably separate the lesion mucous membrane part P5.

Since the electrode portion 46 can be moved in the intersection direction E simply by the operation of the lever 61 of the operation portion 55 of the ESD device 3, the lesion mucous membrane part P5 can be reliably separated even when the lesion mucous membrane part P5 has a small size.

The intermediate portion 43 of the wire portion 40 and the observation unit 15 do not overlap each other when viewed in the direction along the center axis C2. As a result, the intermediate portion 43 of the wire portion 40 does not disturb an operation of acquiring a forward image and the forward image can be reliably acquired by the observation unit 15.

The intermediate portion 43 of the wire portion 40 and the illumination unit 14 do not overlap each other when viewed in the direction along the center axis C2. As a result, the intermediate portion 43 of the wire portion 40 does not disturb an operation of emitting illumination light forward from the illumination unit 14 and a front side can be reliably illuminated by the illumination light.

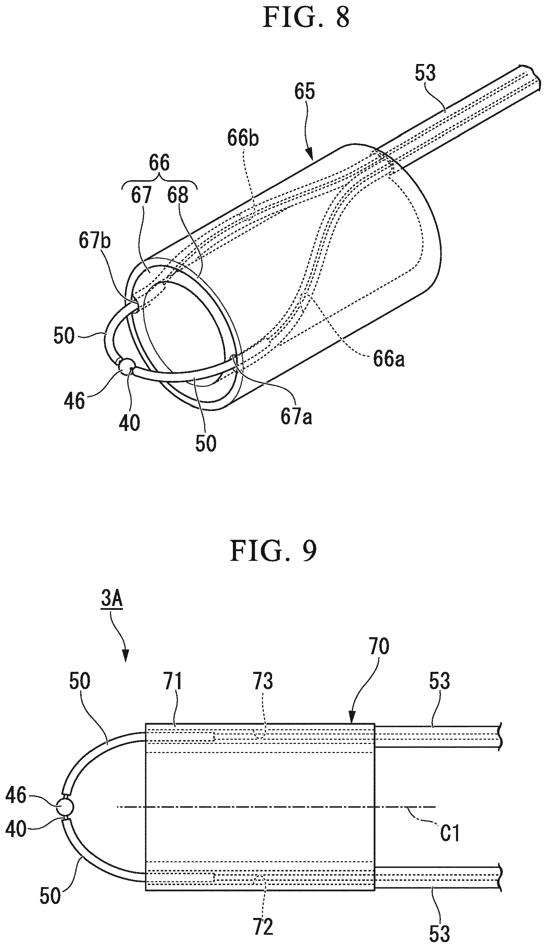

The ESD device 3 of the embodiment can be modified into various forms as will be described below. Figs. 8 to 11 show first to fourth modified examples of the ESD device of the first embodiment.

For example, as in a cap 65 of the first modified example shown in Fig. 8, a cap body 66 may include an inner tube 67 and an outer tube 68 covering an outer circumferential surface of the inner tube 67. The outer circumferential surface of the inner tube 67 is provided with spiral groove portions 67a and 67b corresponding to the penetration holes 31 and 32. When an inner circumferential surface of the outer tube 68 is bonded to the outer circumferential surface of the inner tube 67 by welding or adhesive, a penetration hole 66a is formed by inner circumferential surfaces of the groove portion 67a and the outer tube 68. Similarly, a penetration hole 66b is formed by inner circumferential surfaces of the groove portion 67b and the outer tube 68.

In the first to fourth modified examples, the locking portion 34 is not formed in the cap.

Since the groove portions 67a and 67b of the inner tube 67 are formed at the outer circumferential surface of the inner tube 67, the groove portions 67a and 67b can be easily formed at the inner tube 67 by injection-molding or cutting.

Since the cap 65 has the above-described configuration, the spiral penetration holes 66a and 66b can be easily formed in the cap 65.

A cap 70 of a second modified example is shown in Fig. 9. As in the cap 70 shown in Fig. 9, penetration holes 72 and 73 formed in a cap body 71 may be formed in parallel to the center axis C1 of the cap body 71. In the modified example, an ESD device 3A including the cap 70 includes two sheaths 53. In the two sheaths 53, a lumen of one sheath 53 communicates with the penetration hole 72 and a lumen of the other sheath 53 communicates with the penetration hole 73.

Since the cap 70 has the above-described configuration, the penetration holes 72 and 73 can be easily formed in the cap 70.

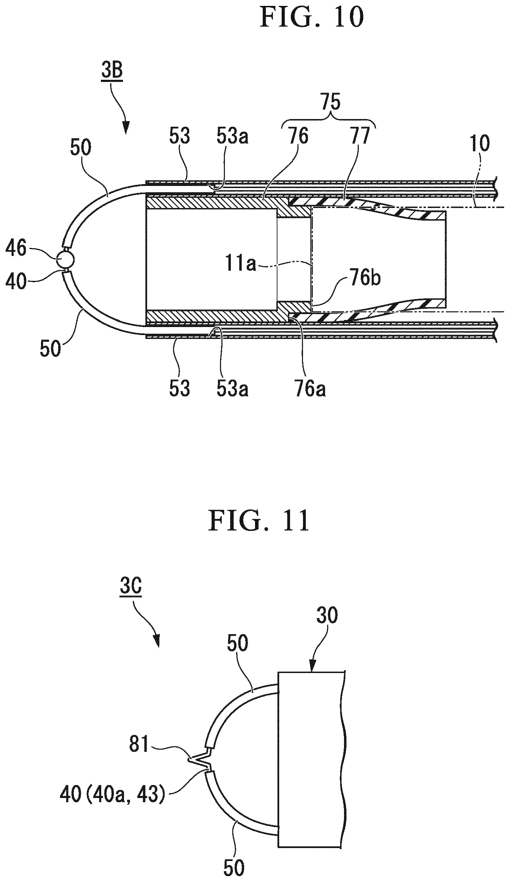

Fig. 10 is a cross-sectional view taken along the direction of the center axis C1 at a distal end of an ESD device 3B of the third modified example. As in the ESD device 3B shown in Fig. 10, the cap 75 may include a cylindrical cap body 76 which is provided at the distal end side and an attachment/detachment portion 77 which is provided at the base end side of the cap body 76.

A stepped portion 76a is formed at a base end of the cap body 76 so that an outer diameter decreases in size. In the modified example, a base end face of the cap body 76 is formed as a locking portion 76b which locks the distal end face 11 a of the insertion portion 10. The cap body 76 can be formed of the same material as that of the cap 30.

The attachment/detachment portion 77 is formed in a cylindrical shape in which an inner diameter thereof is slightly smaller than an outer diameter of the insertion portion 10. A distal end of the attachment/detachment portion 77 is fixed to the stepped portion 76a of the cap body 76 by welding or adhesive. The attachment/detachment portion 77 is formed of a resin material which is softer than the cap body 76.

In the modified example, the cap body 76 and the attachment/detachment portion 77 are not provided with a penetration hole and the distal ends of two sheaths 53 are bonded to an outer circumferential surface of the cap body 76 by welding or adhesive. Each of lumens of the sheaths 53 form penetration holes 53a.

In the cap 75 with the above-described configuration, the insertion portion 10 is inserted into the attachment/detachment portion 77 while the attachment/detachment portion 77 is deformed so that an inner diameter of the attachment/detachment portion 77 increases.

Since the cap 75 has the above-described configuration, the attachment/detachment portion 77 can be brought into contact with an outer circumferential surface of the insertion portion 10.

Fig. 11 shows an ESD device 3C of a fourth modified example. As in the ESD device 3C shown in Fig. 11, an electrode portion 81 may be formed at the intermediate portion 43 of the wire portion 40 by bending the wire 40a forming the wire portion 40.

The electrode portion 81 can be formed, for example, by keeping the wire 40a in a bent state through a mold and heating the mold and the wire 40a in a furnace.

### (Second Embodiment)

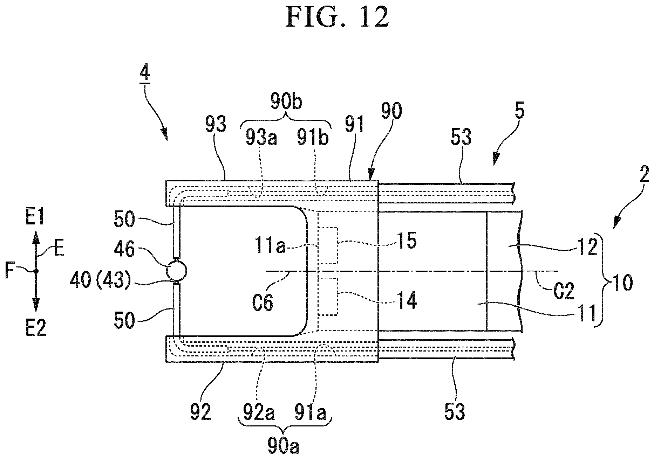

Next, a second embodiment of the present invention will be described with reference to Figs. 12 to 14. Here, the same reference numerals will be given to the same components as those of the above-described embodiment and a description thereof will be omitted. Thus, only differences will be described.

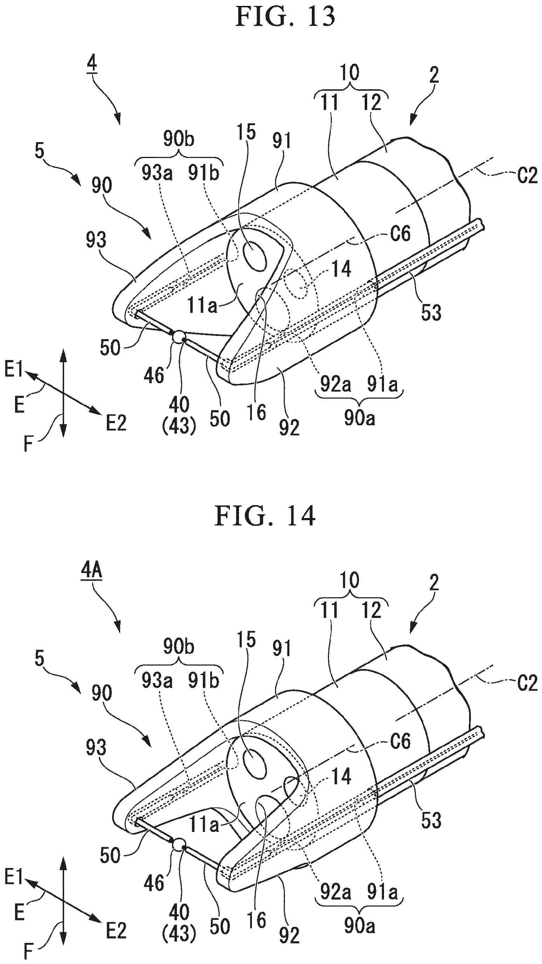

As shown in Figs. 12 and 13, an endoscope system 4 according to the embodiment includes an ESD device 5 instead of the ESD device 3 of the endoscope system 1 according to the first embodiment. The ESD device 5 includes a cap 90 instead of the cap 30 of the ESD device 3.

The cap 90 includes a cylindrical cap body 91 and a pair of arm portions 92 and 93 which protrude toward the distal end side from a distal end face of the cap body 91.

The arm portions 92 and 93 are disposed to be separated from each other in the intersection direction E intersecting a center axis C6 of the cap body 91 with the center axis C6 interposed therebetween.

The cap body 91 is provided with through-holes 91a and 91b extending along the center axis C6 of the cap body 91. The through-holes 91a and 91b are formed to be separated from each other with the center axis C6 interposed therebetween in the radial direction.

As shown in Fig. 13, a length of the arm portion 92 in a width direction F orthogonal to the direction of the center axis C6 and the intersection direction E becomes short as it goes toward the distal end side. The arm portion 92 is provided with a through-hole 92a extending along the center axis C6.

A base end of the through-hole 92a is opened to a base end face of the arm portion 92 and communicates with the through-hole 91 a of the cap body 91. A distal end of the through-hole 92a is bent in the intersection direction E and is opened to a side face of the arm portion 92 near the center axis C6. A penetration hole 90a is formed by the through-hole 91a of the cap body 91 and the through-hole 92a of the arm portion 92.

The arm portion 93 and the arm portion 92 are formed to be plane-symmetric to each other with respect to a symmetry plane (not shown) including the center axis C6. That is, a length of the arm portion 93 in the width direction F becomes short as it goes toward the distal end side. The arm portion 93 is provided with a through-hole 93a extending in the center axis C6.

A base end of the through-hole 93 a is opened to a base end face of the arm portion 93 and communicates with the through-hole 91b of the cap body 91. A distal end of the through-hole 93a is bent in the intersection direction E to be opened to a side face of the arm portion 93 near the center axis C6. A penetration hole 90b is formed by the through-hole 91b of the cap body 91 and the through-hole 93a of the arm portion 93.

The cap body 91 and the arm portions 92 and 93 are formed of the same material as that of the cap 30.

Two sheaths 53 are bonded to the cap body 91. In the two sheaths 53, the lumen of one sheath 53 communicates with the penetration hole 90a and the lumen of the other sheath 53 communicates with the penetration hole 90b.

The wire portion 40 is inserted through the lumen of the sheath 53 and the penetration holes 90a and 90b of the cap 90 to be movable forward and backward. In this example, the intermediate portion 43 of the wire portion 40 extends linearly.

In the embodiment, the insertion portion 10 is inserted into the cap body 91 until the distal end face of the cap body 91 becomes substantially flush with the distal end face 11 a of the insertion portion 10, when the cap 90 is attached to the insertion portion 10.

In the embodiment, an operation of projecting and retracting the electrode portion 46 of the wire portion 40 from and into the cap 90 is not performed and the operation portion 55 is not provided with the slider 57. When the lever 61 of the operation portion 55 is rotated, the electrode portion 46 moves to one side E1 or the other side E2 in the intersection direction E.

Even in the ESD device 5 and the endoscope system 4 with the above-described configuration, the displacement of the field of view of the observation unit 15 can be suppressed even when a tissue dissecting position is displaced in the intersection direction E.

Additionally, in the embodiment, as in an endoscope system 4A of a modified example shown in Fig. 14, a length at the base end side of each of the arm portions 92 and 93 in the width direction F may be shortened.

With such a configuration, a problem in which the illumination light emitted from the illumination unit 14 or the field of view of the observation unit 15 is interrupted by the arm portions 92 and 93 can be suppressed.

### (Third Embodiment)

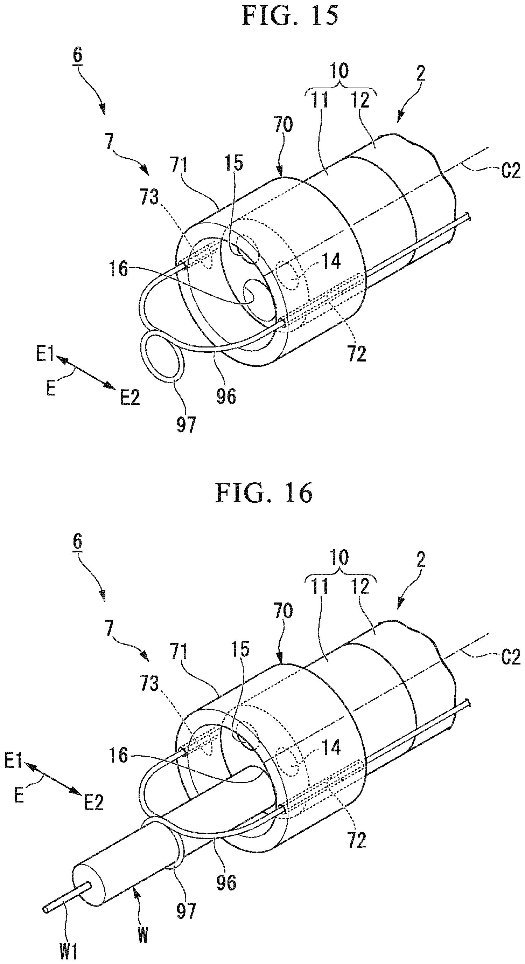

A third embodiment of the present invention will be described with reference to Figs. 15 and 16. Here, the same reference numerals will be given to the same components as those of the above-described embodiments and a description thereof will be omitted. Thus, only differences will be described.

As shown in Fig. 15, an endoscope system 6 according to the embodiment includes the endoscope 2 and an ESD device 7 which is separably attached to the insertion portion 10 of the endoscope 2.

The ESD device 7 includes the above-described cap 70 and an operation wire 96 is inserted through the penetration holes 72 and 73 of the cap 70 to be movable forward and backward. A loop portion 97 which is formed by winding the operation wire 96 in one revolution is formed at a position near the distal end side in relation to the cap 70 in the operation wire 96. The loop portion 97 is formed at a position near the distal end side in relation to the channel 16 so that an inner diameter thereof becomes substantially equal to an inner diameter of the channel 16.

Both ends of the operation wire 96 extend toward the base end side through the penetration holes 72 and 73 and are fixed to the shaft member 60 of the operation portion 55. When the operator rotates the lever 61, the loop portion 97 moves to one side E1 or the other side E2 in the intersection direction E. A high frequency generator is not connected to the operation wire 96.

Next, an operation of the endoscope system 6 with the above-described configuration will be described.

The operator inserts a high-frequency knife W shown in Fig. 16 from the forceps opening 22 of the endoscope 2 into the channel 16. The high-frequency knife W which protrudes from the channel 16 toward the distal end side is inserted through the loop portion 97. A high frequency generator (not shown) is connected to the high-frequency knife W and a high-frequency voltage is applied to a knife portion W1 of the high-frequency knife W.

The knife portion W1 is pushed against a submucosal layer (not shown). When the shaft member 60 is rotated, the knife portion W1 moves in the intersection direction E along with the loop portion 97 to dissect the submucosal layer.

As described above, even in the ESD device 7 and the endoscope system 6 of this embodiment, the same effects as those of the ESD device 3 and the endoscope system 1 of the first embodiment are obtained.

While the first to third embodiments of the present invention have been described with reference to the drawings, a detailed configuration is not limited to the embodiments and also includes various modifications, combinations, and omissions of the embodiments within the spirit of the present invention. Further, it is needless to mention that an appropriate combination of the components of the embodiments can be used.

For example, in the first embodiment and the second embodiment, the wire portion 40 may be formed in a complete loop shape in which the first end portion 41 and the second end portion 42 are connected to each other. In this case, the wire portion 40 is fixed to the shaft member 60 while being wound on the outer circumferential surface at the base end side of the shaft member 60. Similarly, in the third embodiment, the wire portion may be formed in a complete loop shape in which the end at the base end side of the operation wire 96 is connected to the wire portion.

Further, the insulation coating 50 may be formed to cover an entire part other than the electrode portion in the wire portion 40.

While the embodiments of the present invention have been described, the technical scope of the present invention is not limited to the above-described embodiments. That is, the combination of the components of the embodiments can be changed or the components can be modified or omitted without departing from the spirit of the present invention. The present invention is not limited to the description above.

### [Industrial Applicability]

An endoscopic submucosal dissection device which suppresses a displacement of a field of view of an observation portion even when a tissue dissecting position is displaced in a direction intersecting a center axis of an insertion portion and an endoscope system which includes the endoscopic submucosal dissection device can be provided.

### [Reference Signs List]

1, 4, 4A, 6 Endoscope system
2 Endoscope
3, 3A, 3B, 3C, 5, 7 ESD device (endoscopic submucosal dissection device)
10 Insertion portion
14 Illumination unit (illumination portion)
15 Observation unit (observation portion)
31, 32, 53a, 66a, 66b, 72, 73, 90a, 90b Penetration hole (holding portion)
33a, 77 Attachment/detachment portion
33b Mucous membrane support portion
33c Muscular layer support portion
40 Wire portion
41 First end portion
42 Second end portion
43 Intermediate portion
46, 81 Electrode portion
50 Insulation coating (insulation member)
55 Operation portion

## Claims

1. An endoscopic submucosal dissection device comprising:
an attachment/detachment portion that is separably attached to an insertion portion of an endoscope having an observation portion provided at the insertion portion to observe a front side;
a mucous membrane support portion that supports a mucous membrane so that the mucous membrane does not approach a muscular layer;
a muscular layer support portion that supports the muscular layer so that the muscular layer does not approach the mucous membrane;
a wire portion that is formed in a linear shape from a conductive material and is disposed so that an intermediate portion in its longitudinal direction is located at a distal end side in relation to first and second end portions in the longitudinal direction and the intermediate portion is located at the distal end side in relation to the insertion portion;
an insulation member that exposes part of the intermediate portion of the wire portion as an electrode portion and covers an outer circumferential surface of a remaining portion of the intermediate portion of the wire portion; and
an operation portion that is connected to the first and second end portions of the wire portion.

2. The endoscopic submucosal dissection device according to claim 1, further comprising:
a holding portion that holds the wire portion to be movable forward and backward.

3. An endoscope system comprising:
the endoscopic submucosal dissection device according to claim 1; and
the endoscope,
wherein the intermediate portion of the wire portion and the observation portion are disposed so that the intermediate portion of the wire portion and the observation portion do not overlap each other when the attachment/detachment portion of the endoscopic submucosal dissection device is attached to the insertion portion of the endoscope and is viewed in a direction along a center axis of the insertion portion.

4. The endoscope system according to claim 3,
wherein the insertion portion is provided with an illumination portion emitting illumination light forward, and
wherein the intermediate portion of the wire portion and the illumination portion are disposed so that the intermediate portion of the wire portion and the illumination portion do not overlap each other when the attachment/detachment portion of the endoscopic submucosal dissection device is attached to the insertion portion of the endoscope and is viewed in a direction along the center axis.
